Europäisches Patentamt

European Patent Office

Office européen des brevets

(11) Publication number: **0 226 257 B1**

## EUROPEAN PATENT SPECIFICATION

(45) Date of publication of patent specification: **27.01.93**   (51) Int. Cl.⁵: **C01B 33/34**

(21) Application number: **86202269.6**

(22) Date of filing: **16.12.86**

(54) **Synthetic, crystalline, porous material containing silicon oxide, titanium oxide and aluminium oxide.**

(30) Priority: **19.12.85 IT 2329185**

(43) Date of publication of application:
**24.06.87 Bulletin 87/26**

(45) Publication of the grant of the patent:
**27.01.93 Bulletin 93/04**

(84) Designated Contracting States:
**AT BE CH DE ES FR GB GR LI LU NL SE**

(56) References cited:
**EP-A- 0 077 522**
**WO-A-85/04854**
**WO-A-50/04855**
**WO-A-50/04856**
**FR-A- 2 471 950**

(73) Proprietor: **ENIRICERCHE S.p.A.**
**Corso Venezia 16**
**I-20121 Milan(IT)**

Proprietor: **ENICHEM SYNTHESIS S.p.A.**
**Via Ruggero Settimo 55**
**I-90139 Palermo(IT)**

Proprietor: **SNAMPROGETTI S.p.A.**
**Corso Venezia 16**

**I-20121 Milan(IT)**

(72) Inventor: **Bellussi, Giuseppe**
**Via A. Scopo 44**
**I-29100 Piacenza(IT)**
Inventor: **Giusti, Aldo**
**Via Di Meassino 390**
**I-55100 Lucca(IT)**
Inventor: **Esposito, Antonio**
**Via Liberta 70**
**I-20097 San Donato Milanese Milan(IT)**
Inventor: **Buonomo, Franco**
**Via Trento 4**
**I-20097 San Donato Milanese Milan(IT)**

(74) Representative: **Roggero, Sergio et al**
**Ing. Barzanò & Zanardo Milano S.p.A. Via**
**Borgonuovo 10**
**I-20121 Milano(IT)**

Rank Xerox (UK) Business Services

**Description**

This invention relates to a synthetic, crystalline and porous material of zeolite character, containing silicon oxide, titanium oxide and aluminium oxide, and to the process for its preparation.

The material in question is similar to the ZSM-5, an embodiment of US-A-3 702 886, and essentially consisting, in its anhydrous and calcined form, of $M_{2/n}$, $SiO_2$ and $Al_2O_3$, wherein M is an n-valent cation.

Other like materials are known, such as the zeolite disclosed in US-A-4 061 724, which essentially consists, in its anhydrous and calcined form, of SiO2, the zeolite disclosed in FR-A-2 471 950, which is a titanium-containing silica, and the zeolite disclosed in EP-A-0 077 522, which is titanium-containing aluminosilicate.

A novel synthetic zeolite has now been found, that can be defined as a "titanium-aluminium silicalite, which can be employed as a molecular sieve, or an ion-exchanger, or a catalyst in a number of reactions, such as:

cracking, selectoforming, hydrogenation, dehydrogenation, oligomerization, alkylation, isomerization, dehydration of oxygen-containing organic compounds, selective hydroxylation by $H_2O_2$ of organic substrates, oxidation of olefins and hydroxylation of aromatics.

The invention, therefore, provides a synthetic, crystalline and porous material of zeolite character, containing silicon oxide, titanium oxide and aluminium oxide, and having the powder X-ray diffraction spectrum exhibiting the following lines:

| Interplanar distances nm | Rel.Intensities |
|---|---|
| $1,114 \pm 0,010$ | very strong |
| $0,999 \pm 0,010$ | strong |
| $0,974 \pm 0,010$ | medium |
| $0,636 \pm 0,007$ | medium-weak |
| $0,599 \pm 0,007$ | medium-weak |
| $0,426 \pm 0,005$ | medium-weak |
| $0,386 \pm 0,004$ | strong |
| $0,382 \pm 0,004$ | strong |
| $0,375 \pm 0,004$ | strong |
| $0,372 \pm 0,004$ | strong |
| $0,365 \pm 0,004$ | medium |
| $0,305 \pm 0,002$ | medium-weak |
| $0,299 \pm 0,002$ | medium-weak, |

the I.R. spectrum of said material exhibiting at least the following bands:

| Wave number $cm^{-1}$ | Rel.Intensities |
|---|---|
| 1220 to 1230 | weak |
| 1080 to 1110 | strong |
| 965 to 975 | medium-weak |
| 795 to 805 | medium-weak |
| 550 to 560 | medium |
| 450 to 470 | medium-strong |

said material being characterized in that its empirical formula, in its calcined and anhydrous state is:

**$pHAlO_2 .qTiO_2 .SiO_2$**

wherein p is greater than zero and does not exceed 0,050, and q is greater than zero and does not exceed 0,025, the H + of $HAlO_2$ being at least partially replaceable or replaced by cations.

The passage from any cationic form to any other cationic form can be carried out by any conventional exchange procedure.

As outlined above, the material in question is X-ray crystalline.

The X-ray diffraction test has been effected on the powdered material on a diffractometer equipped with an electronic impulse-counting system, with a CuK-alpha radiation: to calculate the intensities, the peaks have been measured and their percentage heights relative to the height of the most intense peak have been calculated.

The X-ray diffraction spectral characteristics are as reported above, and show that the X-ray diffraction is closely akin to that of a ZSM-5 zeolite and like zeolites.

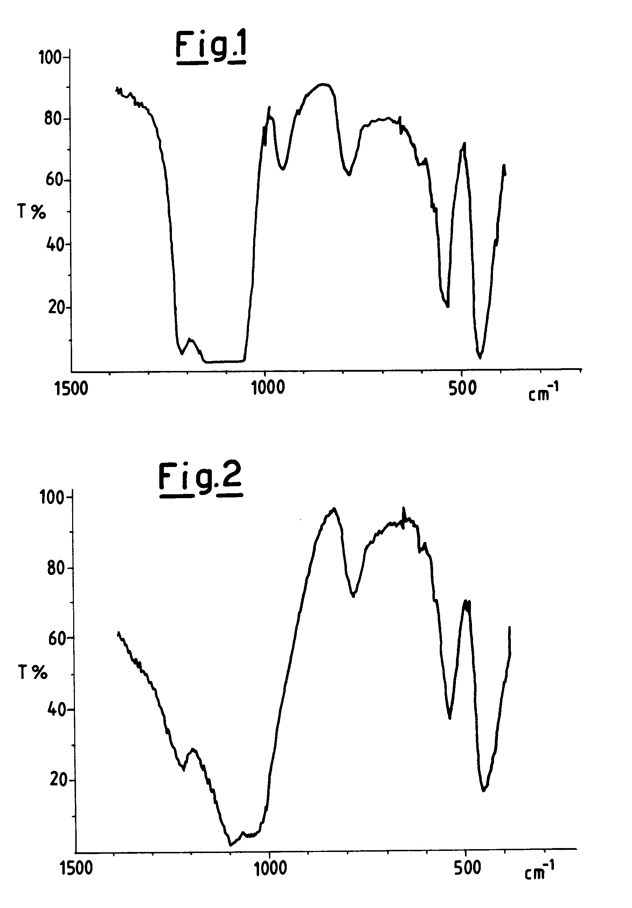

The InfraRed spectrum is as reported above, and is shown in Fig.1 of the accompanying drawings, wherein the wave numbers are plotted on the abscissae, whereas the percentage transmittance values are plotted on the ordinates.

Such an InfraRed spectrum is very closely similar to that of the zeolite as disclosed in FR-A-2 471 950, patent whilst it is considerably different from that of ZSM-5 (or from similar structures), as shown in Figure 2. It can be observed that in this latter spectrum the band at 965-975 $cm^{-1}$, which is characteristic of titanium silicalite of FR-A-2 471 950 and of titanium-aluminum-silicalite, does not appear.

Summarizing, the material as disclosed by ourselves is different from ZSM-5 of US-A-3,702,886, as for both its empirical formula and its I.R. spectrum; relatively to the zeolite of FR-A-2 471 950 as to its empirical formula; and relatively to the zeolite of EP-A-0 077 522 as to its I.R. spectrum.

Furthermore, the use of the subject material of the present invention as a catalyst in the above listed reactions is a further confirmation of the difference of our product relatively to those of the prior art.

In fact, both ZSM-5 of US-A-3,702,886, and the zeolite of EP-A-0 077 522 are used as catalysts in such reactions as dehydrations of oxygenated organic compounds, cracking, selectoforming, hydrogenations and dehydrogenations, oligomerizations, alkylations, isomerizations, but they result inactive in the reactions between organic substrates and $H_2O_2$ (hydroxylation of phenol to diphenols, oxidation of olefins), whilst the zeolite of FR-A-2 471 950 results inactive for the first reactions and active for the last ones; to the contrary, our zeolite is active for all of the above mentioned reactions.

A second object of the present invention is the preparation process for the obtainment of the crystalline, porous, synthetic material as defined above. The invention, therefore, provides also a process for preparing the synthetic, crystalline and porous material as defined in the foregoing, said process comprising the step of reacting, at a temperature of from 120°C to 200°C, at a pH of from 9 to 14 and for a time from 1 h to 5 days a silicon compound, a titanium compound, an aluminium compound and an organic nitrogenous base, in the facultative presence of one or more salts and/or hydroxides of an alkali or alkaline earth metal, the $SiO_2/Al_2O_3$ molar ratio of the reactants being greater than 100, the $SiO_2$ molar ratio of the reactants being greater than 5, and the $M/SiO_2$ molar ratio (wherein M is an alkali metal or an alkaline earth metal) of the reactants being from less than 0,1 to zero.

In the empirical formula of the material, the aluminium has been indicated in the $HAlO_2$ form, to underline that the material is in the $H^+$ form. When speaking of the ratios between the various reactants, we use, for aluminum, the $Al_2O_3$ form, which is the most usual.

The silicon compound is selected from silica gel, silica sol and alkyl silicates, among which, preferably, tetraethyl silicate; the titanium compound is selected from titanium salts, such as, e.g., its halides, and organic titanium derivatives, such as, e.g., alkyltitanates, preferably tetraethyl titanate; the aluminium compound is selected from aluminum salts, such as, e.g., its halides and hydroxides, and its organic derivatives, such as, e.g., alkyl aluminates, preferably isopropyl aluminate.

The nitrogenous organic base can be an alkylammonium hydroxide, preferably tetrapropyl-ammonium hydroxide.

In case tetrapropylammonium hydroxide is used, the $TPA^+/SiO_2$ ratio (wherein TPA = tetrapropylammonium) of the reactants is comprised within the range of from 0.1 to 1, preferably from 0.2 to 0.4.

The reactants are reacted with each other by operating at a temperature of from 100° to 200°C, preferably of from 160° to 180°C, at a pH comprised within the range of from 9 to 14, preferably of from 10 to 12, and for a time period ranging from 1 hour to 5 days, preferably from 3 hours to 10 hours.

For the purpose of better illustrating the meaning of the present invention, some preparation and use examples are given, which in no way are to be considered as being limitative of the invention.

Example 1
------

This example shows the preparation of titanium-aluminum-silicalite.

0.3 g of aluminum isopropoxide is dissolved in 54 g of solution at 18.7% by weight of tetrapropyl-ammonium hydroxide.

In a separate vessel, 2.3 g of tetraethyl-orthotitanate is dissolved in 41.6 g of tetraethyl-silicate and this solution is added to the previously prepared one, with stirring.

The whole mass is heated at 50-60°C, always with stirring, until a single-phase solution is obtained, then 100 cc of water is added.

The obtained solution is charged to an autoclave and is heated, under its autogenous pressure, at 170°c over 4 hours.

The discharged product is centrifuged and washed twice by re-dispersion and centrifuging; it is then dried 1 hour at 120°C, and is then calcined 4 hours at 550°C in the air

The obtained product has an $SiO_2/Al_2O_3$ molar ratio of 98, and an $SiO_2/TiO_2$ molar ratio of 99, and is already in the $H^+$ form.

Examples 2-6

In Table 1 other titanium-aluminum-silicalites are shown, which have been prepared by the same modalities as disclosed in Example 1, by varying the composition of the reactants, and the crystallization times.

The products obtained from Examples 2-5 are in the $H^+$ form, whilst the product obtained from Example 6 is in the $Na^+$ form; it can be turned, by the known processes, into its $H^+$ form.

From the above-reported preparation examples, one can observe that the maximum amounts of titanium and aluminum which can be obtained in the end product are not independent from each other.

The minimum $SiO_2/TiO_2$ ratio which can be obtained in the product is of about 40, and it can be only obtained if $SiO_2/Al_2O_3$ ratio in the product is higher than about 120

In order to obtain $SiO_2/Al_2O_3$ ratios lower than about 120, an increase in $SiO_2/TiO_2$ ratio must be accepted.

At $SiO_2/Al_2O_3$ molar ratios higher than 150 in the reaction mixture (Examples 1-5), the crystallization occurs, and with increasing $SiO_2/Al_2O_3$ ratios in the reactant mixture a decrease in $SiO_2/TiO_2$ ratio and an increase in $SiO_2/Al_2O_3$ ratio occurs in the obtained product.

$SiO_2/TiO_2$ ratio in the obtained product continues to decrease until it reaches its minimum value around 40, which is reached when $SiO_2/Al_2O_3$ in the reaction mixture is of from 300 to 400; further increases of $SiO_2/Al_2O_3$ ratio in the reactant mixture cause only $SiO_2/Al_2O_3$ to increase in the product, whilst $SiO_2/TiO_2$ remains nearly constant.

When the $SiO_2/Al_2O_3$ ratio in the reactant mixture is lower than or equal to 150, adding to the reaction mixture, to the purpose of obtaining the crystallization, salts and/or hydroxides of such cations as tetramethylammonium, ammonium alkali metals or alkali-earth metals (Example 6) is necessary.

These variations cause also an increase in $SiO_2/TiO_2$ ratio in the obtained product.

Examples 7-10

Referring to Table 1, products are shown, which do not have the same characteristics as of the preceding product.

It can be observed from Examples 7, 8 and 9 that, when the molar composition of the reactant mixture is the following:

$SiO_2/AL_2O_3$ = 100-150;

$SiO_2/TiO_2$ = 20;

$TPA^+/SiO_2$ = 0.25;

$H_2O/SiO_2$ = 40;

at a temperature of from 160° to 180°C and under its autogenous pressure, the mixture does not crystallize.

It can be observed from Example 10 that, even if an alkali metal is added ($Na^+/SiO_2$ = 0.04), after 5 hours, the mixture does not crystallized, because the $SiO_2/Al_2O_3$ ratio in the reaction mixture is lower than or equal to 100.

Example 11

Referring to Table 1, the product of Example 11 is shown, which is obtained by starting from the same composition of the reactants of Example 10. It can be observed that after a crystallization time of 96 hours the mixture is crystallized.

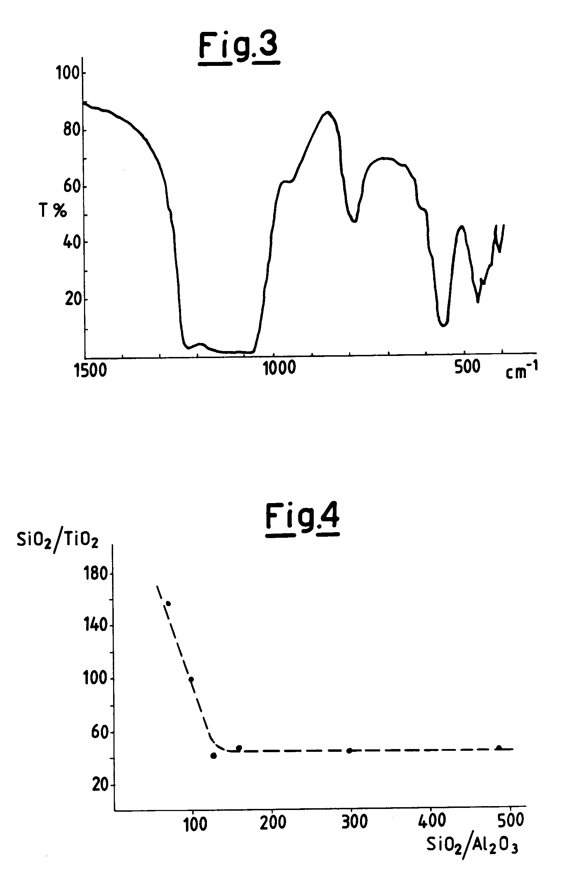

The I.R. spectrum of this product, shown in Figure 3, is not essentially equal to that shown in Figure 1. From the spectrum of Fig. 3, it can be observed, in fact, that the band at 965-975 $cm^{-1}$ is present, but it has a much lower intensity than that represented in the spectrum of Fig. 1, although the product of Example 11 has a higher titanium contents. All of the above allows us to state that the addition of large amounts of

alkali metals ($M^+/SiO_2 \geq 0.04$) in the reaction mass can cause an increase in titanium amount, which can be detected from the chemical analysis of the obtained product, an $SiO_2/TiO_2$ ratio lower than 40 being obtained, but is such case $TiO_2$ is at least partly in a form different from the form it has in the titanium-aluminum-silicalite according to the invention, and such as not to yield the spectrum of Fig. 1, but that of Fig. 3. In Fig. 4, a chart is shown, which sets forth the dependence of the maximum amounts of titanium (shown in the ordinates as $SiO_2/TiO_2$ molar ratio) and of aluminum (shown in the abscissae as $SiO_2/Al_2O_3$ molar ratio) which can be obtained in the end product.

Table 1

| Example | Reaction Mixture Composition | | | | | Crystallization time, hours | Crystallization Temperature, °C | Product Composition | |
|---|---|---|---|---|---|---|---|---|---|
| | $SiO_2/Al_2O_3$ | $SiO_2/TiO_2$ | $Na^+/SiO_2$ | $TPA^+/SiO_2$ | $H_2O/SiO_2$ | | | $SiO_2/Al_2O_3$ | $SiO_2/TiO_2$ |
| 1 | 250 | 20 | - | 0.25 | 40 | 4 | 170 | 98 | 99 |
| 2 | 300 | 20 | - | 0.25 | 40 | 3 1/2 | 170 | 124 | 40 |
| 3 | 400 | 20 | - | 0.25 | 40 | 3 | 170 | 161 | 46 |
| 4 | 800 | 20 | - | 0.25 | 40 | 3 | 170 | 297 | 44 |
| 5 | 1200 | 20 | - | 0.25 | 40 | 3 | 170 | 462 | 43 |
| 6 | 150 | 20 | 0.005 | 0.25 | 40 | 4 | 170 | 65 | 155 |
| 7 | 150 | 20 | - | 0.25 | 40 | 5 | 170 | no crystallization | |
| 8 | 150 | 20 | - | 0.25 | 40 | 72 | 170 | no crystallization | |
| 9 | 100 | 20 | - | 0.25 | 40 | 5 | 170 | no crystallization | |
| 10 | 100 | 20 | 0.04 | 0.25 | 40 | 5 | 170 | no crystallization | |
| 11 | 100 | 20 | 0.04 | 0.25 | 40 | 96 | 170 | 45 | 18 |

Example 12

To a tubular steel reactor, 1.04 g is charged of catalyst of 1mm - 420 mm particle size (18-40 mesh), prepared according to Example 2. The reactor is placed inside and electrical oven and is gradually heated up to the reaction temperature, with a stream of dimethyl ether being flowed through it. The gaseous reaction products are analyzed by in-line chromatography after the liquid products being condensed in a bath kept at 0-5°C. These latter are separately weighed and analyzed, always by chromatographic way. The conversion and selectivity are computed according to the hereunder shown equations:

$$Conversion = \frac{(DME_{in} - DME_{out})}{(DME_{in})}$$

$$Selectivity = \frac{(mol\ of\ i\ product)}{(DME_{in} - DME_{out})}$$

(DME = dimethyl ether; in = incoming; out = outgoing).
    The reaction conditions and the results obtained are gathered in Table 2.

Table_2

| T | (°C) | 320 | 342 |
|---|---|---|---|
| p | (atm) | 1 | 1 |
| GHSV | (h$^{-1}$) | 800 | 1600 |
| Run hours | | 4 | 4 |
| DME Conversion (%) | | 96.2 | 99.6 |

Product_Composition (% by weight - $H_2O$, DME excluded)

| | | | |
|---|---|---|---|
| $CH_3OH$ | | 15.9 | 2.7 |
| $CH_4$ | | 8.4 | 0.2 |
| $C_2H_4$ | | 6.4 | 3.8 |
| $C_2H_6$ | | 0.01 | 0.04 |
| $C_3H_6$ | | 2.3 | 1.5 |
| $C_3H_8$ | | 0.7 | 0.8 |
| $\Sigma C_4$ | | 3.0 | 3.4 |
| $\Sigma C_5$ | | 0.9 | 0.6 |
| $\Sigma C_6^+$ | | 70.3 | 86.9 |

Example 13

    To a small, 250-cc glass flask, there are charged, in the order as shown: phenol, 56.7 g; water, 8.4 g; acetone, 13.5 g; catalyst, prepared as in Example 2, 3 g.

7

The mixture is heated at 100°C with stirring and under reflux, then, under the same conditions, within a 45 minutes time, 10.5 g of 35% (weight/volume) $H_2O_2$ is added dropwise.

After 55 minutes from the beginning of the addition, all of $H_2O_2$ has been converted and the products are analyzed by gas-chromatography.

A yields of diphenols of

obtained diphenol mols = 0.08019

charged $H_2O_2$ mol = 0.099

is obtained.

The residual amount of $H_2O_2$ is converted into pitches and $O_2$. On formed diphenols, ortho/para ratio is 1.18.

## Claims
## Claims for the following Contracting States : BE, CH, DE, FR, GB, GR, LI, LU, NL, SE

1. A synthetic, crystalline and porous material of zeolite character, containing silicon oxide, titanium oxide and aluminium oxide and having the powder X-ray diffraction spectrum exhibiting the following lines:

| Interplanar distances nm | Rel.Intensities |
|---|---|
| $1,114 \pm 0,010$ | very strong |
| $0,999 \pm 0,010$ | strong |
| $0,974 \pm 0,010$ | medium |
| $0,636 \pm 0,007$ | medium-weak |
| $0,599 \pm 0,007$ | medium-weak |
| $0,426 \pm 0,005$ | medium-weak |
| $0,386 \pm 0,004$ | strong |
| $0,382 \pm 0,004$ | strong |
| $0,375 \pm 0,004$ | strong |
| $0,372 \pm 0,004$ | strong |
| $0,365 \pm 0,004$ | medium |
| $0,305 \pm 0,002$ | medium-weak |
| $0,299 \pm 0,002$ | medium-weak, |

the I.R. spectrum of said material exhibiting at least the following bands:

| Wave number $cm^{-1}$ | Rel.Intensities |
|---|---|
| 1220 to 1230 | weak |
| 1080 to 1110 | strong |
| 965 to 975 | medium-weak |
| 795 to 805 | medium-weak |
| 550 to 560 | medium |
| 450 to 470 | medium-strong |

said material being characterized in that its empirical formula, in its calcined and anhydrous state is:

**pHAlO$_2$ .qTiO$_2$ .SiO$_2$**

wherein **p** is greater than zero and does not exceed 0,050, and **q** is greater than zero and does not exceed 0,025, the H + of HAlO$_2$ being at least partially replaceable or replaced by cations.

2. Process for preparing the synthetic, crystalline and porous material as defined in Claim 1, comprising the step of reacting, at a temperature of from 120°C to 200°C, at a pH of from 9 to 14 and for a time of from 1 h to 5 days a silicon compound, a titanium compound, an aluminium compound and an organic nitrogenous base, in the facultative presence of one or more salts and/or hydroxides of an alkali or alkaline earth metal, the $SiO_2/Al_2O_3$ molar ratio of the reactants being greater than 100, the $SiO_2/TiO_2$ molar ratio of the reactants being greater than 5, and the $M/SiO_2$ molar ratio (wherein M is

EP 0 226 257 B1

an alkali metal or an alkaline earth metal) of the reactants being from less than 0,1 to zero.

3. Process according to Claim 2, wherein the $H_2O/SiO_2$ molar ratio of the reactants is from 10 to 100.

4. Process according to Claim 2, wherein the $SiO_2/Al_2O_3$ molar ratio is from 300 to 400, the $SiO_2/TiO_2$ molar ratio of the reactants is from 15 to 25, the $H_2O/SiO_2$ molar ratio of the reactants is from 30 to 50, and the $M/SiO_2$ molar ratio of the reactants is zero.

5. Process according to Claim 2, wherein the silicon compound is selected from silica gel, silica sol and the alkyl silicates, the titanium compound is selected from the salts of organic titanium compounds, and the aluminium compound is selected from the aluminium salts and the organic aluminium compounds.

6. Process according to to Claim 5, wherein the alkyl silicate is tetraethyl silicate.

7. Process according to Claim 5, wherein the titanium salts is a titanium halide.

8. Process according to Claim 5, wherein the organic titanium compound is an alkyl titanate.

9. Process according to Claim 8, wherein the alkyl titanate is tetraethyltitanate.

10. Process according to Claim 5, wherein the aluminium salt is an aluminium halide or an aluminium hydroxide.

11. Process according to Claim 5, wherein the organic aluminium compound is an alkyl aluminate.

12. Process according to Claim 11, wherein the alkyl aluminate is isopropyl aluminate.

13. Process according to Claim 2, wherein the nitrogenous base is an alkylammonium hydroxide.

14. Process according to Claim 13, wherein the alkylammonium hydroxide is tetrapropylammonium hydroxide.

15. Process according to Claim 2, wherein the reaction is carried out at a temperature of from 160°C to 180°C, at a pH of from 10 to 12, and for a time of from 3 h to 10 h.

16. Process according to Claim 14, wherein the $TPA^+/SiO_2$ molar ratio of the reactants, where $TPA^+$ is the tetrapropylammonium ion, is from 0,1 to 1.

17. Process according to Claim 14, wherein the $TPA^+/SiO_2$ ratio of the reactants is from 0,2 to 0,4.

**Claims for the following Contracting States : AT, ES**

1. Process for preparing a synthetic, crystalline and porous material of zeolite character, containing silicon oxide, titanium oxide and aluminium oxide and having the powder X-ray diffraction spectrum exhibiting the following lines:

| Interplanar distance nm | Rel.Intensities |
|---|---|
| $1,114 \pm 0,010$ | very strong |
| $0,999 \pm 0,010$ | strong |
| $0,974 \pm 0,010$ | medium |
| $0,636 \pm 0,007$ | medium-weak |
| $0,599 \pm 0,007$ | medium-weak |
| $0,426 \pm 0,005$ | medium-weak |
| $0,386 \pm 0,004$ | strong |
| $0,382 \pm 0,004$ | strong |
| $0,375 \pm 0,004$ | strong |
| $0,372 \pm 0,004$ | strong |
| $0,369 \pm 0,004$ | medium |
| $0,305 \pm 0,002$ | medium-weak |
| $0,299 \pm 0,002$ | medium-weak, |

the I.R. spectrum of said material exhibiting at least the following bands:

| Wave number $cm^{-1}$ | Rel.Intensities |
|---|---|
| 1220 to 1230 | weak |
| 1080 to 1110 | strong |
| 965 to 975 | medium-weak |
| 795 to 805 | medium-weak |
| 550 to 560 | medium |
| 450 to 470 | medium-strong |

said material having, in its calcined and anhydrous state, the empirical formula:

**pHAlO$_2$ .qTiO$_2$ SiO$_2$**

wherein **p** is greater than zero and does not exceed 0,050, and **q** is greater than zero and does not exceed 0,025, the H+ of HAlO$_2$ being at least partially replaceable or replaced by cations, comprising the step of reacting, at a temperature of from 120°C to 200°C, at a pH of from 9 to 14 and for a time of from 1 h to 5 days a silicon compound, a titanium compound, an aluminium compound and an organic nitrogenous base, in the facultative presence of one or more salts and/or hydroxides of an alkali or alkaline earth metal, the $SiO_2/Al_2O_3$ molar ratio of the reactants being greater than 100, the $SiO_2/TiO_2$ molar ratio of the reactants being greater than 5, and the $M/SiO_2$ molar ratio (wherein M is an alkali metal or an alkaline earth metal) of the reactants being from less than 0,1 to zero.

2. Process according to Claim 1, wherein the $H_2O/SiO_2$ molar ratio of the reactants is from 10 to 100.

3. Process according to Claim 1, wherein the $SiO_2/Al_2O_3$ molar ratio is from 300 to 400, the $SiO_2/TiO_2$ molar ratio of the reactants is from 15 to 25, the $H_2O/SiO_2$ molar ratio of the reactants is from 30 to 50, and the $M/SiO_2$ molar ratio of the reactants is zero.

4. Process according to Claim 1, wherein the silicon compound is selected from silica gel , silica sol and the alkyl silicates , the titanium compound is selcted from the salts of organic titanium compounds, and the aluminium compound is selected from the aluminium salts and the organic aluminium compounds.

5. Process according to to Claim 4, wherein the alkyl silicate is tetraethyl silicate.

6. Process according to Claim 4, wherein the titanium salts is a titanium halide.

7. Process according to Claim 4, wherein the organic titanium compound is an alkyl titanate.

8. Process according to Claim 7, wherein the alkyl titanate is tetraethyltitanate.

**9.** Process according to Claim 4, wherein the aluminium salt is an aluminium halide or an aluminium hydroxide.

**10.** Process according to Claim 4, wherein the organic aluminium compound is an alkyl aluminate.

**11.** Process according to Claim 10, wherein the alkyl aluminate is isopropyl aluminate.

**12.** Process according to Claim 1, wherein the nitrogenous base is an alkylammonium hydroxide.

**13.** Process according to Claim 12, wherein the alkylammonium hydroxide is tetrapropylammonium hydroxide.

**14.** Process according to Claim 1, wherein the reaction is carried out at a temperature of from $160°C$ to $180°C$, at a pH of from 10 to 12, and for a time of from 3 h to 10 h.

**15.** Process according to Claim 13, wherein the $TPA^+/SiO_2$ molar ratio of the reactants, where $TPA^+$ is the tetrapropylammonium ion, is from 0,1 to 1.

**16.** Process according to Claim 15, wherein the $TPA^+/SiO_2$ ratio of the reactants is from 0,2 to 0,4.

**Patentansprüche**
**Patentansprüche für folgende Vertragsstaaten : BE, CH, DE, FR, GB, GR, LI, LU, NL, SE**

**1.** Synthetisches, kristallines, poröses Material mit Zeolithcharakter und mit einem Gehalt an Siliziumoxid, Titanoxid und aluminiumoxid, welches im Röntgen-Pulverbeugungsspektrum die folgenden Linien aufweist:

| Netzebenenabstand nm | rel. Intensität |
|---|---|
| $1,114 \pm 0,010$ | sehr stark |
| $0,999 \pm 0,010$ | stark |
| $0,9741 \pm 0,010$ | mittel |
| $0,636 \pm 0,007$ | mittel-schwach |
| $0,599 \pm 0,007$ | mittel-schwach |
| $0,426 \pm 0,005$ | mittel-schwach |
| $0,386 \pm 0,004$ | stark |
| $0,382 \pm 0,004$ | stark |
| $0,375 \pm 0,004$ | stark |
| $0,372 \pm 0,004$ | stark |
| $0,365 \pm 0,004$ | mittel |
| $0,305 \pm 0,002$ | mittel-schwach |
| $0,299 \pm 0,002$ | mittel-schwach, |

im I.R.-Spektrum wenigstens die folgenden Banden zeigt:

| Wellenzahl $cm^{-1}$ | rel. Intensität |
|---|---|
| 1.220 bis 1.230 | schwach |
| 1.080 bis 1.110 | stark |
| 965 bis 975 | mittel-schwach |
| 795 bis 805 | mittel-schwach |
| 550 bis 560 | mittel |
| 450 bis 470 | mittel-stark, |

und welches Material dadurch gekennzeichnet ist, daß es in seinem calzinierten und wasserfreien Zustand der empirischen Formel:

**pHAlO$_2$ . qTiO$_2$ . SiO$_2$**

entspricht, worin p größer als null ist und den Wert 0,050 nicht überschreitet und q größer als 0 ist und den Wert 0,025 nicht überschreitet, wobei H+ von HAlO$_2$ wenigstens teilweise durch Kationen ersetzbar oder ersetzt ist.

2. Verfahren zur Herstelltung des synthetischen kristallinen porösen Materials nach Anspruch 1, umfassend die Stufe des Umsetzens, bei einer Temperatur von 120°C bis 200°C, bei einem pH-Wert von 9 bis 14 und während einer Zeitdauer von einer Stunde bis zu 5 Tagen, einer Siliziumverbindung, einer Titanverbindung, einer Aluminiumverbindung und einer organischen stickstoffhältigen Base, gegebenenfalls in Anwesenheit eines oder mehrerer Salze und/oder Hydroxide eines Alkali- oder Erdalkalimetalles, wobei das SiO$_2$/Al$_2$O$_3$-Molverhältnis der Reaktanten größer als 100 ist, das SiO$_2$/TiO$_2$-Molverhältnis der Reaktanten größer als 5 ist und das M/SiO$_2$-Molverhältnis (worin M ein Alkalimetall oder ein Erdalkalimetall bedeutet) der Reaktanten von kleiner als 0,1 bis null beträgt.

3. Verfahren nach Anspruch 2, worin das H$_2$O/SiO$_2$-Molverhältnis der Reaktanten von 10 bis 100 beträgt.

4. Verfahren nach Anspruch 2, worin das SiO$_2$/Al$_2$O$_3$-Molverhältnis von 300 bis 400 beträgt, das SiO$_2$/TiO$_2$-Molverhältnis der Reaktanten von 15 bis 25 beträgt, das H$_2$O/SiO$_2$-Molverhältnis der Reaktanten von 30 bis 50 beträgt und das M/SiO$_2$-Molverhältnis der Reaktanten 0 ausmacht.

5. Verfahren nach Anspruch 2, worin die Siliziumverbindung unter Silicagel, Silicasol und Alkylsilikaten ausgewählt ist, die Titanverbindung unter den Salzen und organischen Titanverbindungen ausgewählt ist und die Aluminiumverbindung unter den Aluminiumsalzen und den organischen Aluminiumverbindungen ausgewählt ist.

6. Verfahren nach Anspruch 5, worin das Alkylsilikat Tetraethylsilikat ist.

7. Verfahren nach Anspruch 5, worin das Titansalz ein Titanhalogenid ist,

8. Verfahren nach Anspruch 5, worin die organische Titanverbindung ein Alkyl titanat ist.

9. Verfahren nach Anspruch 8, worin das Alkyltitanat Tetraethyltitanat ist.

10. Verfahren nach Anspruch 5, worin das Aluminiumsalz ein Aluminiumhalogenid oder ein Aluminiumhydroxid ist.

11. Verfahren nach Anspruch 5, worin die organische Aluminiumverbindung ein Alkylaluminat ist.

12. Verfahren nach Anspruch 11, worin das Alkylaluminat Isopropylaluminat ist.

13. Verfahren nach Anspruch 2, worin die stickstoffhältige Base ein Alkylammoniumhydroxid ist.

14. Verfahren nach Anspruch 13, worin das Alkylammoniumhydroxid Tetrapropylammoniumhydroxid ist.

15. Verfahren nach Anspruch 2, worin die Umsetzung bei einer Temperatur von 160 bis 180°C, einem pH-Wert von 10 bis 12 und während einer Zeit von 3 Stunden bis 10 Stunden ausgeführt wird.

16. Verfahren nach Anspruch 14, worin das TPA$^+$/SiO$_2$-Molverhältnis der Reaktanten 0,1 bis 1 beträgt, worin TPA$^+$ das Tetrapropylammoniumion bezeichnet.

17. Verfahren nach Anspruch 14, worin das TPA$^+$/SiO$_2$-Verhältnis der Reaktanten 0,2 bis 0,4 beträgt.

**Patentansprüche für folgende Vertragsstaaten : AT, ES**

1. Verfahren zur Herstellung eines synthetischen,kristallinen und porösen Materials mit Zeolithcharakter und mit einem Gehalt an Siliziumoxid, Titanoxid und Aluminiumoxid, welches im Röntgen-Pulverbeugungsspektrum die folgenden Linien aufweist:

| Netzebenenabstand nm | rel. Intensität |
|---|---|
| 1,114 ± 0,010 | sehr stark |
| 0,999 ± 0,010 | stark |
| 0,974 ± 0,010 | mittel |
| 0,636 ± 0,007 | mittel-schwach |
| 0,599 ± 0,007 | mittel-schwach |
| 0,426 ± 0,005 | mittel-schwach |
| 0,386 ± 0,004 | stark |
| 0,382 ± 0,004 | stark |
| 0,375 ± 0,004 | stark |
| 0,372 ± 0,004 | stark |
| 0,365 ± 0,004 | mittel |
| 0,305 ± 0,002 | mittel-schwach |
| 0,299 ± 0,002 | mittel-schwach, |

im I.R.-Spektrum wenigstens die folgenden Banden zeigt:

| Wellenzahl cm$^{-1}$ | rel. Intensität |
|---|---|
| 1.220 bis 1.230 | schwach |
| 1.080 bis 1.110 | stark |
| 965 bis 975 | mittel-schwach |
| 795 bis 805 | mittel-schwach |
| 550 bis 560 | mittel |
| 450 bis 470 | mittel-stark, |

welches Material in seinem calcinierten und wasserfreien Zustand der empirischen Formel:

**pHAlO$_2$ . qTiO$_2$ . SiO$_2$**

entspricht, worin **p** größer als null ist und den Wert 0,050 nicht überschreitet und **q** größer als 0 ist und den Wert 0,025 nicht überschreitet, wobei H+ von HAlO$_2$ wenigstens teilweise durch Kationen ersetzbar oder ersetzt ist, welches Verfahren die Stufe des Umsetzens, bei einer Temperatur von 120°C bis 200°C, bei einem pH-Wert von 9 bis 14 und während einer Zeitdauer von einer Stunde bis zu 5 Tagen, einer Siliziumverbindung, einer Titanverbindung, einer Aluminiumverbindung und einer organischen stickstoffhältigen Base, gegebenenfalls in Anwesenheit eines oder mehrerer Sälze und/oder Hydroxide eines Alkali- oder Erdalkslimetalles, wobei des SiO$_2$/Al$_2$O$_3$-Molverhältnis der Reaktanten größer als 100 ist, das SiO$_2$/TiO$_2$-Molverhältnis der Reäktanten größer als 5 ist und des M/SiO$_2$-Molverhältnis (worin M ein Alkalimetall oder ein Erdalkalimetall bedeutet) der Reaktanten von kleiner als 0,1 bis null beträgt, umfäßt,

2. Verfahren nach Anspruch 1, worin des H$_2$O/SiO$_2$-Molverhältnis der Reaktanten von 10 bis 100 beträgt.

3. Verfahren nach Anspruch 1, worin das SiO$_2$/Al$_2$O$_3$-Molverhältnis von 300 bis 400 beträgt, des SiO$_2$/TiO$_2$-Molverhältnis der Reaktanten von 15 bis 25 beträgt, das H$_2$O/SiO$_2$-Molverhältnis der Reaktanten von 30 bis 50 beträgt und das M/SiO$_2$-Molverhältnis der Reaktanten 0 ausmacht.

4. Verfahren nach Anspruch 1, worin die Siliziumverbindung unter Silicagel, Silicasol und Alkylsilikaten ausgewählt ist, die Titanverbindung unter den Salzen und organischen Titanverbindungen ausgewählt ist und die Aluminiumverbindung unter den Aluminiumsalzen und den organischen Aluminiumverbindungen ausgewählt ist.

5. Verfahren nach Anspruch 4, worin das Alkylsilikat Tetraethylsilikat ist.

6. Verfahren nach Anspruch 4, worin das Titansalz ein Titanhalogenid ist.

**7.** Verfahren nach Anspruch 4, worin die organische Titanverbindung ein Alkyltitanat ist.

**8.** Verfahren nach Anspruch 7, worin das Alkyltitanat Tetraethyltitanat ist.

**9.** Verfahren nach Anspruch 4, worin das Aluminiumsalz ein Aluminiumhalogenid oder ein Aluminiumhydroxid ist.

**10.** Verfahren nach Anspruch 4, worin die organische Aluminiumverbindung ein Alkylaluminat ist.

**11.** Verfahren nach Anspruch 10, worin das Alkylaluminat Isopropylaluminat ist.

**12.** Verfahren nach Anspruch 1, worin die stickstoffhältige Base ein Alkylammoniumhydroxid ist.

**13.** Verfahren nach Anspruch 12, worin das Alkylammoniumhydroxid Tetrapropylammoniumhydroxid ist.

**14.** Verfahren nach Anspruch 1, worin die Umsetzung bei einer Temperatur von 160 bis 180°C, einem pH-Wert von 10 bis 12 und während einer Zeit von 3 Stunden bis 10 Stunden ausgeführt wird.

**15.** Verfahren nach Anspruch 13, worin des $TPA^+/SiO_2$-Molverhältnis der Reaktanten 0,1 bis 1 beträgt, worin $TPA^+$ das Tetrapropylammoniumion bezeichnet.

**16.** Verfahren nach Anspruch 15, worin des $TPA^+/SiO_2$-Verhältnis der Reaktanten 0,2 bis 0,4 beträgt.

**Revendications**

**Revendications pour les Etats contractants suivants : BE, CR, DE, FR, GB, GR, LI, LU, NL, SE**

**1.** Matériau synthétique, cristallin et poreux, de nature zéolithique, contenant de l'oxyde de silicium, de l'oxyde de titane et de l'oxyde d'aluminium et présentant, à l'état de poudre, un spectre de diffraction de rayons X présentant les raies suivantes :

| Distances interplanaires nm | Intensités relatives |
|---|---|
| 1,114 ± 0,010 | très forte |
| 01999 ± 0,010 | forte |
| 0,974 ± 0,010 | moyenne |
| 0,636 ± 0,007 | moyenne-faible |
| 0,599 ± 0,007 | moyenne-faible |
| 0,426 ± 0,005 | moyenne-faible |
| 0,386 ± 0,004 | forte |
| 0,382 ± 0,004 | forte |
| 0,375 ± 0,004 | forte |
| 0,372 ± 0,004 | forte |
| 0,365 ± 0,004 | moyenne |
| 0,305 ± 0,002 | moyenne-faible |
| 0,299 ± 0,002 | moyenne-faible |

le spectre infrarouge dudit matériau présentant au moins les bandes suivantes :

| Nombre d'onde $cm^{-1}$ | Intensités relatives |
|---|---|
| 1220 à 1230 | faible |
| 1080 à 1110 | forte |
| 965 à 975 | moyenne-faible |
| 795 à 805 | moyenne-faible |
| 550 à 560 | moyenne |
| 450 à 470 | moyenne-forte |

ledit matériau étant caractérisé en ce que sa formule empirique, à l'état calciné et anhydre, est la suivante :

$pHAlO_2 . qTiO_2 . SiO_2$

dans laquelle p est supérieur à zéro et ne dépasse pas 0,050 et q est supérieur à zéro et ne dépasse pas 0,025, les ions $H^+$ de $HAlO_2$ étant au moins partiellement remplaçables ou remplacés par des cations.

2. Procédé de préparation du matériau synthétique cristallin et poreux défini dans la revendication 1, comprenant l'étape consistant à faire réagir, à une température de 120°C à 200°C, à un pH de 9 à 14 et pendant une durée de 1 heure à 5 jours, un composé du silicium, un composé du titane, un composé de l'aluminium et une base organique azotée, en la présence facultative d'un ou de plusieurs sels et/ou hydroxydes de métal alcalin ou alcalino-terreux, le rapport molaire $SiO_2/Al_2O_3$ des réactifs étant supérieur à 100, le rapport molaire $SiO_2/TiO_2$ des réactifs étant supérieur à 5, et le rapport molaire $M/SiO_2$ des réactifs, M représentant un métal alcalin ou alcalino-terreux, valant de moins de 0,1 à zéro.

3. Procédé conforme à la revendication 2, dans lequel le rapport molaire $H_2O/SiO_2$ des réactifs vaut de 10 à 100.

4. Procédé conforme à la revendication 2, dans lequel le rapport molaire $SiO_2/Al_2O_3$ vaut de 300 à 400, le rapport molaire $SiO_2/TiO_2$ des réactifs vaut de 15 à 25, le rapport molaire $H_2O/SiO_2$ des réactifs vaut de 30 à 50 et le rapport molaire $M/SiO_2$ des réactifs vaut zéro.

5. Procédé conforme à la revendication 2, dans lequel le composé du silicium est choisi parmi un gel de silice, un sol de silice et les silicates d'alkyle, le composé du titane est choisi parmi les sels de titane et les composés organiques du titane, et le composé de l'aluminium est choisi parmi les sels d'aluminium et les composés organiques de l'aluminium.

6. Procédé conforme à la revendication 5, dans lequel le silicate d'alkyle est du silicate de tétraéthyle.

7. Procédé conforme à la revendication 5, dans lequel le sel de titane est un halogénure de titane.

8. Procédé conforme à la revendication 5, dans lequel le composé organique du titane est un titanate d'alkyle.

9. Procédé conforme à la revendication 8, dans lequel le titanate d'alkyle est du titanate de tétraéthyle.

10. Procédé conforme à la revendication 5, dans lequel le sel d'aluminium est un halogénure d'aluminium ou un hydroxyde d'aluminium.

11. Procédé conforme à la revendication 5, dans lequel le composé organique d'aluminium est un aluminate d'alkyle.

12. Procédé conforme à la revendication 11, dans lequel l'aluminate d'alkyle est l'aluminate d'isopropyle.

13. Procédé conforme à la revendication 2, dans lequel la base azotée est un hydroxyde d'alkylammonium.

14. Procédé conforme à la revendication 13, dans lequel l'hydroxyde d'alkylammonium est de l'hydroxyde de tétrapropylammonium.

15. Procédé conforme à la revendication 2, dans lequel la réaction est effectuée à une température de 160°C à 180°C, à un pH de 10 à 12 et pendant une durée de 3 heures à 10 heures.

16. Procédé conforme à la revendication 14, dans lequel le rapport molaire $TPA^+/SiO_2$ des réactifs, $TPA^+$ représentant l'ion tétrapropylammonium, vaut de 0,1 à 1.

EP 0 226 257 B1

**17.** Procédé conforme à la revendication 14, dans lequel le rapport TPA$^+$/SiO$_2$ des réactifs vaut de 0,2 à 0,4.

**Revendications pour les Etats contractants suivants : AT, ES**

**1.** Procédé de préparation d'un matériau synthétique, cristallin et poreux, de nature zéolithique, contenant de l'oxyde de silicium, de l'oxyde de titane et de l'oxyde d'aluminium et présentant, à l'état de poudre, un spectre de diffraction de rayons X présentant les raies suivantes :

| Distances interplanaires nm | Intensités relatives |
|---|---|
| 1,114 ± 0,010 | très forte |
| 0,999 ± 0,010 | forte |
| 0,974 ± 0,010 | moyenne |
| 0,636 ± 0,007 | moyenne-faible |
| 0,599 ± 0,007 | moyenne-faible |
| 0,426 ± 0,005 | moyenne-faible |
| 0,386 ± 0,004 | forte |
| 0,382 ± 0,004 | forte |
| 0,375 ± 0,004 | forte |
| 0,372 ± 0,004 | forte |
| 0,365 ± 0,004 | moyenne |
| 0,305 ± 0,002 | moyenne-faible |
| 0,299 ± 0,002 | moyenne-faible |

le spectre infrarouge dudit matériau présentant au moins les bandes suivantes :

| Nombre d'onde cm$^{-1}$ | Intensités relatives |
|---|---|
| 1220 à 1230 | faible |
| 1080 à 1110 | forte |
| 965 à 975 | moyenne-faible |
| 795 à 805 | moyenne-faible |
| 550 à 560 | moyenne |
| 450 à 470 | moyenne-forte |

ledit matériau présentant, à l'état calciné et anhydre, la formule empirique suivante :

pHAlO$_2$ . qTiO$_2$ . SiO$_2$

dans laquelle p est supérieur à zéro et ne dépasse pas 0,050 et q est supérieur à zéro et ne dépasse pas 0,025, les ions H$^+$ de HAlO$_2$ étant au moins partiellement remplaçables ou remplacés par des cations,

ledit procédé comprenant l'étape consistant à faire réagir, à une température de 120°C à 200°C, à un pH de 9 à 14 et pendant une durée de 1 heure à 5 jours, un composé du silicium, un composé du titane, un composé de l'aluminium et une base organique azotée, en la présence facultative d'un ou de plusieurs sels et/ou hydroxydes de métal alcalin ou alcalino-terreux, le rapport molaire SiO$_2$/Al$_2$O$_3$ des réactifs étant supérieur à 100, le rapport molaire SiO$_2$/TiO$_2$ des réactifs étant supérieur à 5, et le rapport molaire M/SiO$_2$ des réactifs, M représentant un métal alcalin ou alcalino-terreux, valant de moins de 0,1 à zéro.

**2.** Procédé conforme à la revendication 1, dans lequel le rapport molaire H$_2$O/SiO$_2$ des réactifs vaut de 10 à 100.

**3.** Procédé conforme à la revendication 1, dans lequel le rapport molaire SiO$_2$/Al$_2$O$_3$ vaut de 300 à 400, le rapport molaire SiO$_2$/TiO$_2$ des réactifs vaut de 15 à 25, le rapport molaire H$_2$O/SiO$_2$ des réactifs vaut de 30 à 50 et le rapport molaire M/SiO$_2$ des réactifs vaut zéro.

**4.** Procédé conforme à la revendication 1, dans lequel le composé du silicium est choisi parmi un gel de silice, un sol de silice et les silicates d'alkyle, le composé du titane est choisi parmi les sels de titane et les composés organiques du titane, et le composé de l'aluminium est choisi parmi les sels d'aluminium et les composés organiques de l'aluminium.

**5.** Procédé conforme à la revendication 4, dans lequel le silicate d'alkyle est du silicate de tétraéthyle.

**6.** Procédé conforme à la revendication 4, dans lequel le sel de titane est un halogénure de titane.

**7.** Procédé conforme à la revendication 4, dans lequel le composé organique du titane est un titanate d'alkyle.

**8.** Procédé conforme à la revendication 7, dans lequel le titanate d'alkyle est du titanate de tétraéthyle.

**9.** Procédé conforme à la revendication 4, dans lequel le sel d'aluminium est un halogénure d'aluminium ou un hydroxyde d'aluminium.

**10.** Procédé conforme à la revendication 4, dans lequel le composé organique d'aluminium est un aluminate d'alkyle.

**11.** Procédé conforme à la revendication 10, dans lequel l'aluminate d'alkyle est l'aluminate d'isopropyle.

**12.** Procédé conforme à la revendication 1, dans lequel la base azotée est un hydroryde d'aluminium.

**13.** Procédé conforme à la revendication 12, dans lequel l'hydroxyde d'alkylammonium est de l'hydroxyde de tétrapropylammonium.

**14.** Procédé conforme à la revendication 1, dans lequel la réaction est effectuée à une température de 160°C à 180°C, à un pH de 10 à 12 et pendant une durée de 3 heures à 10 heures.

**15.** Procédé conforme à la revendication 13, dans lequel le rapport molaire $TPA^+/SiO_2$ des réactifs, $TPA^+$ représentant l'ion tétrapropylammonium, vaut de 0,1 à 1.

**16.** Procédé conforme à la revendication 15, dans lequel le rapport $TPA^+/SiO_2$ des réactifs vaut de 0,2 à 0,4.

Fig.1

Fig.2

# Fig.3

# Fig.4